(19) Europäisches Patentamt
European Patent Office
Office européen des brevets

(11) Veröffentlichungsnummer: **0 118 093**
**A1**

(12) # EUROPÄISCHE PATENTANMELDUNG

(21) Anmeldenummer: **84102049.8**

(22) Anmeldetag: **28.02.84**

(51) Int. Cl.$^3$: **C 07 C 127/22**
A 01 N 47/34, C 07 D 213/75
C 07 D 239/42, C 07 D 251/46
C 07 D 251/22, C 07 D 307/14

(30) Priorität: **08.03.83 JP 36671/83**

(43) Veröffentlichungstag der Anmeldung:
**12.09.84 Patentblatt 84/37**

(84) Benannte Vertragsstaaten:
**BE CH DE FR GB IT LI NL**

(71) Anmelder: **NIHON TOKUSHU NOYAKU SEIZO K.K.**
**No.4, 2-chome, Nihonbashi Honcho Chuo-ku**
**Tokyo 103(JP)**

(72) Erfinder: **Yamada, Yasuo**
**5-15-9, Nishihirayama**
**Hino-shi Tokyo(JP)**

(72) Erfinder: **Saito, Junichi**
**3-7-12, Osawa**
**Mitaka-shi Tokyo(JP)**

(72) Erfinder: **Gotoh, Toshio**
**20-1-304, 5-chome, Seishin**
**Sagamihara-shi Kanagawa-ken(JP)**

(72) Erfinder: **Katsumata, Osamu**
**438, Midori-cho**
**Hachioji-shi Tokyo(JP)**

(72) Erfinder: **Sakawa, Shinji**
**1-14-13, Tamadaira**
**Hino-shi Tokyo(JP)**

(74) Vertreter: **Schumacher, Günter, Dr. et al,**
**c/o Bayer AG Konzernverwaltung RP Patentabteilung**
**D-5090 Leverkusen 1 Bayerwerk(DE)**

(54) **Trichloroacryloylharnstoff-Derivate, Verfahren zu ihrer Herstellung und ihre Verwendung als Fungizide für Landwirtschaft und Gartenbau.**

(57) Neue Trichloracryloylharnstoff-Derivate der allgemeinen Formel (I)

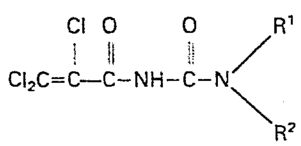

in welcher
$R^1$ für Wasserstoff, Alkyl, Alkenyl, Alkinyl, Cycloalkyl oder für durch Cyano-Gruppen substituiertes Alkyl steht und
$R^2$ für Wasserstoff, Alkyl, Alkenyl, Alkinyl, Cycloalkylalkyl, Cyanoalkyl, Tetrahydrofurfuryl, gegebenenfalls substituiertes Cycloalkyl, gegebenenfalls substituiertes Aryl, gegebenenfalls substituiertes Aralkyl oder für eine gegebenenfalls substituierte heterocyclische Gruppe steht
sowie ihre Verwendung als Fungizide.
Die neuen Trichloracryloylharnstoff-Derivate können hergestellt werden, indem man Trichloracryloyl-isocyanat mit geeigneten Aminen umsetzt.

EP 0 118 093 A1

NIHON TOKUSHU NOYAKU SEIZO K.K. / Tokyo, Japan

# Trichloroacryloylharnstoff-Derivate, Verfahren zu ihrer Herstellung und ihre Verwendung als Fungizide für Landwirtschaft und Gartenbau

Die vorliegende Erfindung betrifft neue Trichloroacryloylharnstoff-Derivate, ein Verfahren zu ihrer Herstellung und ihre Verwendung als Fungizide.

Aus "Pflanzenschutz und Schädlingsbekämpfung" ("Plant Protection and Pesticides"), S. 127, von K.H. Büchel, erschienen im Georg Thieme Verlag Stuttgart (1977) und aus "Pesticide Manual", Seite 40, herausgegeben von dem British Crop Protection Council, 5. Aufl., (Jan. 1977), ist bekannt, daß S-Benzyl-O,O-diisopropylphosphorothioat (Verbindung A) als fungizides Mittel gegen phytopathogene Pilze eingesetzt wird. Diese Verbindung wird als im Handel erhältliches Produkt im Pflanzenschutz anerkannt und läßt sich in Form von Standard-Präparaten beschreiben.

Weiterhin sind 2,3-Dihalogenalkanoyl-harnstoffe mit fungiziden Eigenschaften bekannt (US-PS 4 101 575).

Die vorliegende Erfindung betrifft neue Trichloroacryloylharnstoff-Derivate der nachstehenden allgemeinen Formel (I)

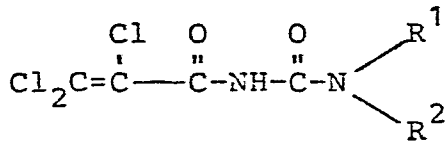

(I) ,

in der

$R_1$ für ein Wasserstoff-Atom, eine Alkyl-Gruppe, eine niedere Alkenyl-Gruppe, eine niedere Alkinyl-Gruppe, eine Cycloalkyl-Gruppe oder eine cyano-substituierte niedere Alkyl-Gruppe und

$R_2$ für ein Wasserstoff-Atom, eine Alkyl-Gruppe, eine niedere Alkenyl-Gruppe, eine niedere Alkinyl-Gruppe, eine Cycloalkyl-alkyl-Gruppe, eine cyano-substituierte niedere Alkyl-Gruppe, eine Tetrahydrofurfuryl-Gruppe, eine gegebenenfalls substituierte Cycloalkyl-Gruppe, ein gegebenenfalls substituiertes Aryl, eine gegebenenfalls substituierte Aralkyl-Gruppe oder eine gegebenenfalls substituierte heterocyclische Gruppe

stehen.

Die Trichloroacryloylharnstoff-Derivate der allgemeinen Formel (I) können mittels des folgenden Verfahrens hergestellt werden, auf das sich die vorliegende Erfindung ebenfalls erstreckt.

Ein Verfahren zur Herstellung der Trichloroacryloylharnstoff-Derivate der allgemeinen Formel (I) umfaßt die Umsetzung von Trichloroacryloylisocyanat der Formel

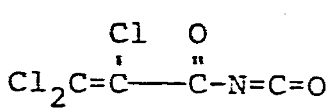

(II)

mit einem Amin der nachstehenden allgemeinen Formel

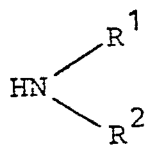

(III)

,

worin $R^1$ und $R^2$ die im Vorstehenden angegebenen Bedeutungen haben, gegebenenfalls in einem Verdünnungsmittel.

Die neuen Trichloroacryloylharnstoff-Derivate der vorliegenden Erfindung sind dadurch gekennzeichnet, daß sie in ihrer durch die Formel (I) bezeichneten chemischen Struktur eine Harnstoff-Struktur als Grundskelett aufweisen und die Trichloroacryloyl-Gruppe an eines ihrer Stickstoff-Atome und $R^1$ und $R^2$ an das andere Stickstoff-Atom gebunden sind. Es wurde gefunden, daß die Verbindungen der vorliegenden Erfindung aufgrund dieser neuen Struktur eine ausgezeichnete fungizide Aktivität erreichen.

Überraschenderweise entfalten die Verbindungen der Formel (I) gemäß der vorliegenden Erfindung dadurch eine beträchtlich stärkere Wirkung als die aus dem Stand der Technik bekannten Verbindungen, die unter dem Gesichtspunkt ihrer Wirkung eng verwandte Verbindungen sind.

Von den neuen Trichloroacryloylharnstoff-Derivaten der Formel (I) sind diejenigen bevorzugt, in denen

$R_1$ für ein Wasserstoff-Atom, eine Alkyl-Gruppe mit 1 bis 8 Kohlenstoff-Atomen, eine niedere Alkenyl-Gruppe mit 2 bis 6 Kohlenstoff-Atomen, eine niedere Alkinyl-Gruppe mit 3 bis 6 Kohlenstoff-Atomen, eine Cycloalkyl-Gruppe mit 5 bis 7 Kohlenstoff-Atomen oder eine cyano-substituierte niedere Alkyl-Gruppe mit 1 bis 8 Kohlenstoff-Atomen steht und

$R_2$ für ein Wasserstoff-Atom, eine Alkyl-Gruppe mit 1 bis 8 Kohlenstoff-Atomen, eine niedere Alkenyl-Gruppe mit 2 bis 6 Kohlenstoff-Atomen, eine niedere Alkinyl-Gruppe mit 3 bis 6 Kohlenstoff-Atomen, eine Cyclopropylmethyl-Gruppe, eine cyano-substituierte niedere Alkyl-Gruppe mit 1 bis 8 Kohlenstoff-Atomen in der Alkyl-Struktureinheit, eine Tetrahydrofurfuryl-Gruppe, eine Cycloalkyl-Gruppe mit 5 bis 7 Kohlenstoff-Atomen, die substituiert sein kann durch wenigstens einen Substituenten ausgewählt aus der aus niederen Alkyl-Gruppen, Trifluoromethyl-Gruppen und niederen Alkinyl-Gruppen bestehenden Klasse, eine Aryl-Gruppe mit 6 bis 10 Kohlenstoff-Atomen, die substituiert sein kann durch wenigstens einen Substituenten ausgewählt aus der aus Halogen-Atomen, niederen Alkyl-Gruppen, niederen Alkoxy-Gruppen, niederen Alkylthio-Gruppen, Trihalogenomethyl-Gruppen, Cyano-Gruppen, Nitro-Gruppen, Phenyl-Gruppen, Cyclopropyl-Gruppen, Phenoxy-Gruppen, Niederalkylthioniederalkyl-Gruppen und nitro-substituierten Phenylthio-Gruppen, eine Aralkyl-Gruppe mit 6 bis 10 Kohlenstoff-Atomen in der Aryl-Struktureinheit und 1 bis 3 Kohlenstoff-Atomen im Alkyl-Teil, die in

dem Aryl-Teil substituiert sein kann durch wenigstens einen Substituenten ausgewählt aus der gleichen Klasse der Substituenten wie im Falle der vorstehenden Aryl-Gruppe und die in dem Alkyl-Teil durch Halogen substituiert sein kann, oder eine 6-gliedrige heterocyclische Gruppe mit 1 bis 3 Stickstoff-Atomen, die substituiert sein kann durch wenigstens einen Substituenten ausgewählt aus der gleichen Klasse der Substituenten wie im Falle der vorstehenden Aryl-Gruppe, steht.

Besonders bevorzugte Trichloroacryloylharnstoff-Derivate der Formel (I) sind diejenigen, in denen

$R_1$ für ein Wasserstoff-Atom, eine Alkyl-Gruppe mit 1 bis 8 Kohlenstoff-Atomen wie Methyl, Ethyl, Propyl, Isopropyl, n-(iso, sec- oder tert)Butyl, n-Pentyl, n-Hexyl, n-Heptyl und n-Octyl, eine niedere Alkenyl-Gruppe mit 2 bis 4 Kohlenstoff-Atomen wie Vinyl, Allyl, 1-Propenyl und 1-Butenyl, eine niedere Alkinyl-Gruppe mit 2 oder 3 Kohlenstoff-Atomen wie Ethinyl und 1-Propinyl, eine Cycloalkyl-Gruppe mit 5 bis 7 Kohlenstoff-Atomen wie Cyclopentyl, Cyclohexyl und Cycloheptyl und die gleiche niedere Alkyl-Gruppe wie oben, die cyanosubstituiert ist, wie eine 2-Cyanoethyl-Gruppe, steht und

$R_2$ für ein Wasserstoff-Atom, eine Alkyl-Gruppe mit 1 bis 8 Kohlenstoff-Atomen, eine niedere Alkenyl-Gruppe mit 2 bis 4 Kohlenstoff-Atomen wie vorstehend, die gleichen niederen Alkinyl-Gruppen mit 2 oder 3 Kohlenstoff-Atomen wie vorstehend, eine Cyclopropylmethyl-Gruppe, die gleichen niederen Alkyl-Gruppen mit 1 bis 8 Kohlenstoff-Atomen in

der Alkyl-Struktureinheit wie vorstehend, die cyano-substituiert sind, eine Tetrahydrofurfuryl-Gruppe, eine Cycloalkyl-Gruppe mit 5 bis 7 Kohlenstoff-Atomen, die substituiert sein kann, wobei der Substituent wenigstens ein Glied ausgewählt aus der aus den gleichen niederen Alkyl-Gruppen mit 1 bis 8 Kohlenstoff-Atomen wie vorstehend, einer Trifluoromethyl-Gruppe und den gleichen niederen Alkinyl-Gruppen mit 2 oder 3 Kohlenstoff-Atomen wie vorstehend bestehenden Klasse, Aryl-Gruppen mit 6 bis 10 Kohlenstoff-Atomen wie Phenyl oder Naphthyl, die substituiert sein können, Aralkyl-Gruppen mit 6 bis 10 Kohlenstoff-Atomen in dem Aryl-Teil und 1 bis 3 Kohlenstoff-Atomen im Alkyl-Teil, die substituiert sein können, und 6-gliedrige heterocyclische Gruppen mit 1 bis 3 Stickstoff-Atomen, die substituiert sein können. Die Substituenten, die die Aryl-, Aralkyl- oder heterocyclische Gruppe aufweisen können, bestehen aus wenigstens einem Substituenten ausgewählt aus der aus Halogen-Atomen wie Fluor, Chlor, Brom und Iod, den gleichen niederen Alkyl-Gruppen mit 1 bis 8 Kohlenstoff-Atomen wie vorstehend, niederen Alkoxy-Gruppen mit 1 bis 8 Kohlenstoff-Atomen, niederen Alkylthio-Gruppen mit 1 bis 8 Kohlenstoff-Atomen, Trihalogenomethyl-Gruppen mit den gleichen Halogen-Atomen wie vorstehend, einer Cyano-Gruppe, einer Nitro-Gruppe, einer Phenyl-Gruppe, einer Cyclopropyl-Gruppe, einer Phenoxy-Gruppe, Niederalkylthio-niederalkyl-Gruppen mit 1 bis 8 Kohlenstoff-Atomen pro Alkyl-Struktureinheit und einer nitro-substituierten Phenylthio-Gruppe bestehenden Klasse.

Der Alkyl-Teil des Aralkyl-Substituenten kann durch 1 bis 5 Halogen-Atome substituiert sein.

Wenn beispielsweise Trichloroacryloyl-isocyanat und 4-Trifluoromethylanilin als Ausgangsstoffe eingesetzt werden, wird der Ablauf der Reaktion gemäß der vorliegenden Erfindung zur Erzeugung der Verbindungen der Formel (I) durch die folgende Gleichung erläutert:

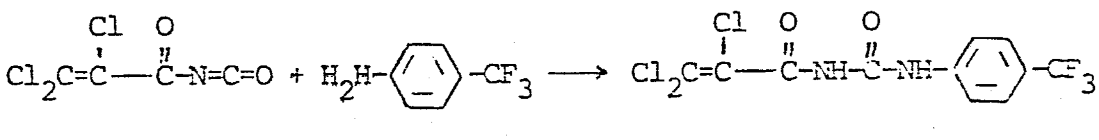

In dem Verfahren zur Erzeugung der Verbindung der vorliegenden Erfindung, das durch das obige Reaktionsschema dargestellt ist, ist die Ausgangs-Verbindung der Formel (II) Trichloroacryloyl-isocyanat. Dieses Ausgangsmaterial für die Herstellung der Verbindungen gemäß der vorliegenden Erfindung kann mittels eines bekannten Verfahrens synthetisiert werden, bei dem Trichloroacrylamid mit Oxalylchlorid umgesetzt wird, wie dies nachstehend schematisch dargestellt ist:

$$Cl_2C{=}\overset{\overset{Cl}{|}}{C} - \overset{\overset{O}{\|}}{C}{-}NH_2 + (COCl)_2$$

$$\longrightarrow Cl_2C{=}\overset{\overset{Cl}{|}}{C} - \overset{\overset{O}{\|}}{C}{-}N{=}C{=}O + 2HCl + CO$$

Die in dem Verfahren gemäß der vorliegenden Erfindung eingesetzten Amine der Formel (III) sind bereits bekannte Verbindungen, und diese können mittels an sich bekannter Verfahren hergestellt werden.

Zu speziellen Beispielen für das Ausgangsmaterial der allgemeinen Formel (III) zählen Anilin, 4-Chloroanilin, 2-Chloroanilin, 2-Fluoroanilin, 3,5-Dichloroanilin, 2,4,6-Trichloroanilin, 4-Chloro-o-toluidin, 4-Nitro-2,6-diiodoanilin, 4-Trifluoromethylanilin, 2-Trifluoromethylanilin, 3,5-Bis-trifluoromethylanilin, N-Methyl-3,5-bis-trifluoromethylanilin, 2-Nitroanilin, 2-Cyanoanilin, 2-Methoxy-5-nitroanilin, 2-Methoxyanilin, 3-Isopropoxyanilin, 4-Phenoxyanilin, 2-Phenoxyanilin, 2,6-Xylidin, N-Methyl-2,3-xylidin, 6-Ethyl-o-toluidin, 2,6-Diisopropylanilin, N-Methyl-2,6-xylidin, 2-Methylthioanilin, 2-Methylthiomethylanilin, 4-(4-Nitrophenylthio)-anilin, 2-Phenylanilin, α-Naphthylamin, 5-Chloro-2-pyridylamin, 2-Chloro-3-pyridylamin, 3,5-Dichloro-2-pyridylamin, 3,5-Dibromo-2-pyridylamin, 5-Chloro-3-methyl-2-pyridylamin, 3,5-Dichloro-6-methyl-2-pyridylamin, 3-Methyl-2-pyridylamin, 6-Methyl-2-pyridylamin, 6-Methoxy-3-pyridylamin, 3-Nitro-2-pyridylamin, 2-Pyrimidinylamin, 4,6-Dichloro-2-pyrimidinylamin, 5-tert-Butyl-2-pyrimidinylamin, 5-Chloro-4,6-dimethyl-2-pyrimidinylamin, 4,6-Dimethoxy-1,3,5-triazin-2-ylamin, 4,6-Dicyclopropyl-1,3,5-triazin-2-ylamin, Benzylamin, Phenethylamin, 4-Chlorobenzylamin, 3,4-Dichlorobenzylamin, 4-Methylbenzylamin, 4-Isopropylbenzylamin, 4-Methoxybenzylamin, N-Methyl-α-trichloromethyl-benzylamin, N-Cyclopentyl-4-chloro-benzylamin, N-sec-Butyl-4-chlorobenzylamin, Diallylamin, 2-Propinylamin, Di-(2-propinyl)-amin, Di-(2-cyanoethyl)-amin, Dioctylamin, Cyclopropylmethylamin, Tetrahydrofurylamin, 2-Methylcyclohexylamin, 3-Trifluoromethylcyclohexylamin, 1-Ethinyl-

cyclohexylamin, 1,3,5-Triazin-2-ylamin, 2-Pyridylamin, Cyclopentylamin und Cycloheptylamin.

Die erfindungsgemäße Reaktion des Trichloroacryloylisocyanats der Formel (II) mit den Aminen der Formel (III) kann in einem Lösungsmittel oder Verdünnungsmittel durchgeführt werden. Zu diesem Zweck können sämtliche inerten Lösungsmittel und Verdünnungsmittel verwendet werden.

Beispiele für solche Lösungsmittel oder Verdünnungsmittel sind aliphatische, alicyclische und aromatische Kohlenwasserstoffe (gegebenenfalls chlorierte) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Di-isopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Ester wie Ethylacetat und Amylacetat; Säureamide wie Dimethylformamid und Dimethylacetamid; Sulfone und Sulfoxide wie Dimethylsulfoxid und Sulfolan; sowie Basen wie Pyridin.

Das Verfahren gemäß der vorliegenden Erfindung kann innerhalb eines weiten Temperatur-Bereichs durchgeführt werden, gewöhnlich bei einer Temperatur zwischen etwa -20°C und dem Siedepunkt der Mischung, günstigerweise zwischen etwa 0°C und etwa 100°C. Zweckmäßigerweise wird die Reaktion unter atmosphärischem Druck durchgeführt, jedoch ist es möglich, unter erhöhtem oder vermindertem Druck zu arbeiten.

Die aktiven Verbindungen gemäß der vorliegenden Erfindung zeigen eine starke mikrobizide Wirkung.

Es wurde gefunden, daß eine solche fungizide Aktivität sehr anwendungsfreundlich und genau für Anwendungen in Landwirtschaft und Gartenbau genutzt werden können und daß sie sich besonders für die Bekämpfung der Brusone-Krankheit von Reis eignet.

Es wurde weiterhin gefunden, daß die Verbindungen der vorliegenden Erfindung neben der der im Vorstehenden angegebenen Struktur zuzuschreibenden fungiziden Aktivität Eindringvermögen besitzen und infolgedessen eine hervorragende fungizide Wirksamkeit gegen die Brusone-Krankheit von Reis mit Tiefenwirkung wie im Falle der Anwendung auf das Laub ausüben sowie ebenfalls eine ausgeprägte Bekämpfungswirkung gegen die Brusone-Krankheit von Reis zeigen, wenn sie auf die Oberfläche oder das Innere des Wassers in einem Reisfeld zur Einwirkung gebracht werden.

Es wurde weiterhin gefunden, daß die Verbindungen der vorliegenden Erfindung eine niedrige Toxizität gegenüber warmblütigen Tieren besitzen und in den üblichen Konzentrationen überhaupt keine Phytotoxizität gegen Kulturpflanzen zeigen und infolgedessen in bezug auf die Sicherheit Vorteile bieten.

Die Verbindungen gemäß der vorliegenden Erfindung besitzen ein breites fungizides Spektrum und können in wirksamer Weise gegen verschiedene Pflanzenkrankheiten eingesetzt werden, die beispielsweise durch Archimycetes, Phycomycetes, Ascomycetes, Basidiomycetes und Fungi imperfecti sowie durch Bakterien verursacht werden.

Es wurde weiterhin gefunden, daß diese neuen Verbindungen eine hervorragende Wirkung als Fungizide in Landwirtschaft und Gartenbau zeigen, insbesondere eine ausgezeichnete Bekämpfungswirkung gegen die Brusone-Krankheit von Reis (Pyricularia oryzae).

Als Fungizid für Landwirtschaft und Gartenbau können die Verbindungen gemäß der vorliegenden Erfindung unmittelbar nach Verdünnen mit Wasser oder aber in Form verschiedener Formulierungen eingesetzt werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Einsatz werden diese Präparate entweder unmittelbar oder nach dem Verdünnen mit Wasser auf die gewünschte Konzentration zur Anwendung gebracht. Beispiele für solche Anwendungsformen umfassen emulgierbare Konzentrate, Öl-Präparate, benetzbare Pulver, lösliche Pulver, suspendierende Mittel, Stäubemittel, Granulat, Pulverpräparate, Räuchermittel, Tabletten, Aerosole, Pasten und Kapseln.

Beispiele für die landwirtschaftlich unbedenklichen Hilfsstoffe, auf die hier Bezug genommen wird, sind Verdünnungsmittel (Lösungsmittel, Streckmittel, Träger), oberflächenaktive Mittel (Lösungsvermittler, Emulgatoren, Dispergiermittel, Netzmittel), Stabilisatoren, Haftmittel, Aerosol-Treibmittel und synergistische Mittel.

Beispiele für die Lösungsmittel sind Wasser und organische Lösungsmittel, beispielsweise Kohlenwasserstoffe /¯z.B. n-Hexan, Petrolether, Naphtha, Erdöl-Fraktionen (z.B. Paraffinwachse, Kerosin, Leichtöle, Mittelöle,

Schweröle), Benzol, Toluol und Xylole_7, halogenierte Kohlenwasserstoffe (z.B. Methylenchlorid, Kohlenstofftetrachlorid, Trichloroethylen, Ethylenchlorid, Ethylendibromid, Chlorbenzol und Chloroform), Alkohole (z.B. Methylalkohol, Ethylalkohol, Propylalkohol und Ethylenglycol), Ether (z.B. Diethylether, Ethylenoxid und Dioxan), Alkohol-ether (z.B. Ethylenglycol-monomethylether), Ketone (z.B. Aceton und Isophoron), Ester (z.B. Ethylacetat und Amylacetat), Amide (z.B. Dimethylformamid und Dimethylacetamid) und Sulfoxide (z.B. Dimethylsulfoxid).

Beispiele für die Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z.B. Pyrophyllit, Talkum, Montmorillonit, Beidellit, Vermikulit, Kaolinit und Glimmer), pflanzliche Pulver wie beispielsweise Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für oberflächenaktive Mittel umfassen anionische oberflächenaktive Mittel wie Alkylschwefelsäureester (z.B. Natriumlaurylsulfat), Arylsulfonsäuren (z.B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z.B. Laurylamin, Stearyltrimethylammoniumchlorid

und Alkyldimethylbenzylammoniumchlorid) und Polyoxyethylenalkylamine, nicht-ionische oberflächenaktive Mittel wie Polyoxyethylenglycolether (z.B. Polyoxyethylenalkylarylether und deren Kondensationsprodukte), Polyoxyethylenglycolester (z.B. Polyoxyethylenfettsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel vom Vinylacetat-Typ und Acryl-Haftmittel), Aerosol-Treibmittel (z.B. Trichlorofluoromethan, Dichlorofluoromethan, 1,2,2-Trichloro-1,1,2-trifluoroethan, Chlorbenzol, verflüssigtes Erdgas und niedere Ether), die Verbrennung steuernde Mittel für Räucherpräprate (z.B. Nitrite, Zink-Pulver und Dicyandiamid), Sauerstoff abgebende Mittel (z.B. Chlorate und Bichromate), wirkungsverlängernde Mittel, Dispersions-Stabilisatoren [z.B. Casein, Tragant, Carboxymethylcellulose (CMC) und Polyvinylalkohol (PVA)] und synergistische Mittel.

Die landwirtschaftlichen Fungizide gemäß der vorliegenden Erfindung können etwa 0,1 bis etwa 95 Gew.-%, vorzugsweise etwa 0,5 bis etwa 90 Gew.-% des vorerwähnten aktiven Wirkstoffes enthalten.

Für den praktischen Gebrauch beträgt die geeignete Menge der aktiven Verbindung in den vorgenannten Mitteln in ihren verschiedenen Formen und gebrauchsfertigen Präparaten beispielsweise etwa 0,0001 bis etwa 20 Gew.-%, vorzugsweise etwa 0,005 bis etwa 10 Gew.-%.

Der Gehalt des Wirkstoffs kann in geeigneter Weise je nach der Form des Präparats oder Mittels, dem Verfahren, Zweck, der Zeit und dem Ort seiner Anwendung, dem Zustand des Auftretens einer Pflanzenkrankheit etc. variiert werden.

Erforderlichenfalls können die Verbindungen gemäß der vorliegenden Erfindung weiterhin auch in Kombination mit anderen Agrochemikalien verwendet werden, beispielsweise mit Insektiziden, anderen Fungiziden, Mitiziden, Nematiziden, Anti-Virus-Mitteln, Herbiziden, Pflanzen-Wachstumsregulatoren und Lockstoffen /̄ Organophosphat-Verbindungen, Carbamat-Verbindungen, Dithio-(oder thiol)carbamat-Verbindungen, organischen Chlor-Verbindungen, Dinitro-Verbindungen, organischen Schwefel- oder Metall-Verbindungen, Antibiotika, substituierten Diphenylether-Verbindungen, Harnstoff-Verbindungen und Triazin-Verbindungen_7 und/oder Düngemitteln.

Die den im Vorstehenden bezeichneten Wirkstoff enthaltenden verschiedenartigen Mittel und gebrauchsfertigen Präparate können mittels verschiedener Verfahren zur Anwendung gebracht werden, wie sie allgemein für das Aufbringen von Agrochemikalien gebräuchlich sind, beispielsweise durch Sprühen (Versprühen von Flüssigkeiten, Vernebeln, Zerstäuben, Stäuben, Streuen von Granulat, Wasser-Oberflächenbehandlung, Gießen etc.) Räuchern, Bodenbehandlung (Vermischen mit dem Boden, Streuen, Bedampfen, Gießen etc.), Oberflächenbehandlung (Beschichten, Bändern, Pulverbeschichten, Bedecken etc.) und Eintauchen. Sie können auch mittels des sogenannten Ultra-Low-Volume-Sprühverfahrens aufgebracht werden. Nach diesem Verfahren kann der Wirkstoff sogar in einer Konzentration von 100 % zur Anwendung gelangen.

Die Aufwandmengen pro Flächeneinheit betragen beispielsweise etwa 0,03 bis etwa 10 kg/ha, vorzugsweise etwa 0,3 bis etwa 6 kg/ha. In besonders gelagerten Fällen können oder sollten sogar die Aufwandmengen außerhalb des angegebenen Bereichs liegen.

Die folgenden Beispiele erläutern die vorliegende Erfindung im einzelnen. Es sei jedoch darauf hingewiesen, daß die vorliegende Erfindung nicht allein auf diese speziellen Beispiele beschränkt ist.

Beispiel 1 (Herstellungsbeispiel)

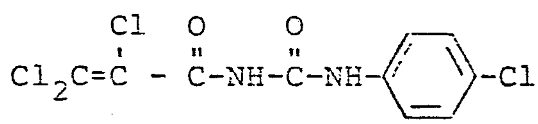

(Verbindung Nr. 1)

1,9 g 4-Chloroanilin wurden in 40 ml Toluol gelöst, und unter Rühren wurden 3,0 g Trichloroacryloylisocyanat bei einer Temperatur unterhalb von 20°C hinzugefügt. Die Lösung wurde über Nacht stehen gelassen, und die erhaltenen Kristalle wurden durch Zugabe von 50 ml n-Hexan gesammelt. Die gesammelten Kristalle wurden aus Aceton/n-Hexan umkristallisiert, wonach 4,0 g des gewünschten N-Trichloroacryloyl-N'-4-chlorophenylharnstoffs in Form weißer Kristalle erhalten wurden. Das Produkt hatte einen Schmelzpunkt von 194°C bis 195°C.

Tabelle 1 enthält die Verbindungen gemäß der vorliegenden Erfindung, die im wesentlichen nach den gleichen Methoden wie vorstehend synthetisiert wurden.

Tabelle 1

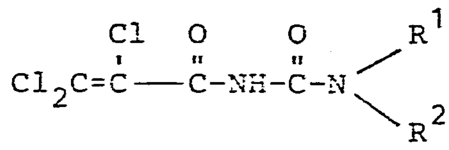

| Verbindung Nr. | $R^1$ | $R^2$ | Physikal. Konstante (Schmp. °C) |
|---|---|---|---|
| 2 | H | 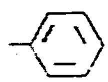 | 169-171 |
| 3 | H | 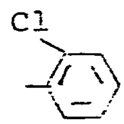 | 177-178 |
| 4 | H | 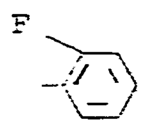 | 170-172 |
| 5 | H | 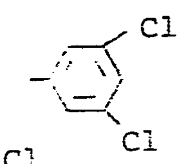 | 187-188 |
| 6 | H | 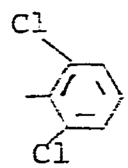 | 191-193.5 |
| 7 | H | 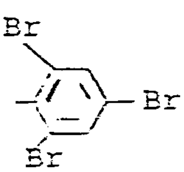 | 218-219 |
| 8 | H | 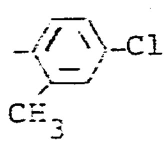 | 178-180 |

Tabelle 1 - Fortsetzung

| Verbindung Nr. | $R^1$ | $R^2$ | Physikal. Konstante (Schmp. °C) |
|---|---|---|---|
| 9 | H | 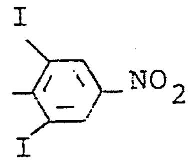 | 258-259 |
| 10 | H | 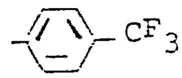 | 184-185 |
| 11 | H | 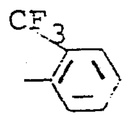 | 117.5-119 |
| 12 | H | 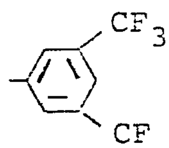 | 136-137 |
| 13 | $-CH_3$ | 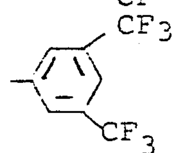 | 145-147 |
| 14 | H | 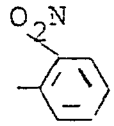 | 183-184 |
| 15 | H | 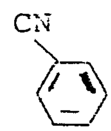 | 181.5-183 |
| 16 | H | 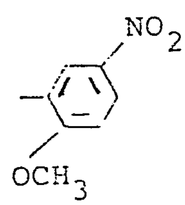 | 240-241 |

Tabelle 1 - Fortsetzung

| Verbindung Nr. | $R^1$ | $R^2$ | Physikal. Konstante (Schmp. °C) |
|---|---|---|---|
| 17 | H | 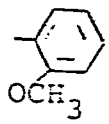 | 177.5-178.5 |
| 18 | H | 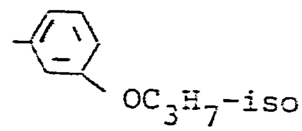 | 102-104 |
| 19 | H | 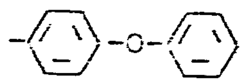 | 120-124 |
| 20 | H | 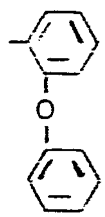 | 157-160 |
| 21 | H | 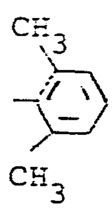 | 184-185 |
| 22 | $-CH_3$ | 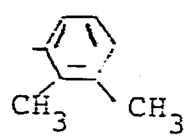 | 141-142 |
| 23 | H | 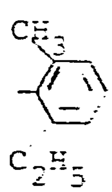 | 147-149 |

Tabelle 1 - Fortsetzung

| Verbindung Nr. | $R^1$ | $R^2$ | Physikal. Konstante (Schmp. °C) |
|---|---|---|---|
| 24 | H | 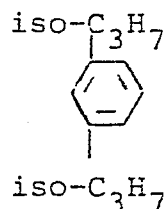 | 221-222 |
| 25 | $-CH_3$ | 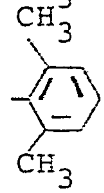 | 127-129 |
| 26 | H | 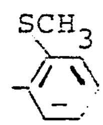 | 128-130 |
| 27 | H | 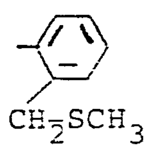 | 129.5-131 |
| 28 | H | 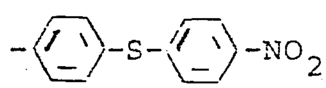 | 140-143 |
| 29 | H | 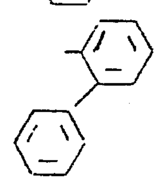 | 159.5-160.5 |
| 30 | H | 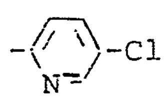 | 190-191 |
| 31 | H | 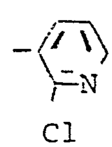 | 183-184 |

Tabelle 1 - Fortsetzung

| Verbindung Nr. | $R^1$ | $R^2$ | Physikal. Konstante (Schmp. °C) |
|---|---|---|---|
| 32 | H | 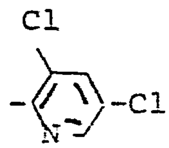 | 162-164 |
| 33 | H | 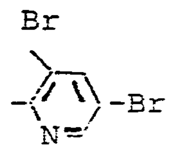 | 147-149 |
| 34 | H | 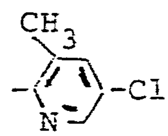 | 182-183 |
| 35 | H | 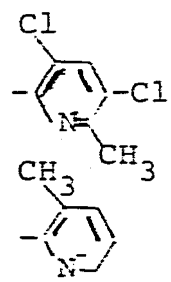 | 170.5-171.5 |
| 36 | H | | 146-149 |
| 37 | H | 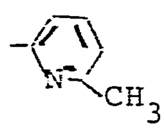 | 121-125 |
| 38 | H | 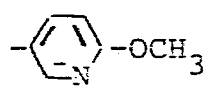 | 173-176 |
| 39 | H | 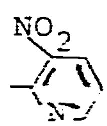 | 189-192 |

Tabelle 1 - Fortsetzung

| Verbindung Nr. | $R^1$ | $R^2$ | Physikal. Konstante (Schmp. °C) |
|---|---|---|---|
| 40 | H | 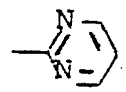 | 242 ( Zers. ) |
| 41 | H | 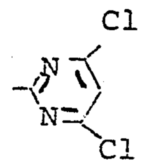 | 193-194 |
| 42 | H | 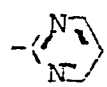 -$C_4H_9$-tert. | 176-177 |
| 43 | H | 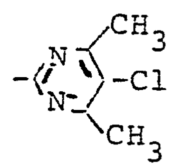 | 192-194 |
| 44 | H | 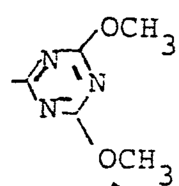 | 210-211 |
| 45 | H | 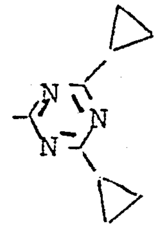 | 226-228 |
| 46 | H | -$CH_2$- 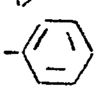 | 135-136 |
| 47 | H | $CH_2$-$CH_2$- 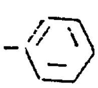 | 117.5-120 |

Tabelle 1 - Fortsetzung

| Verbindung Nr. | $R^1$ | $R^2$ | Physikal. Konstante (Schmp. °C) |
|---|---|---|---|
| 48 | H | 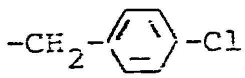 | 156-157 |
| 49 | H | 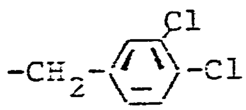 | 150-152 |
| 50 | H | 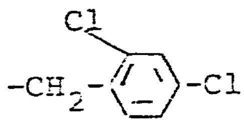 | 120-121 |
| 51 | H | 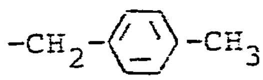 | 149-151 |
| 52 | H | 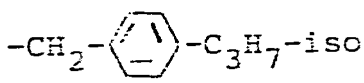 | 135-136 |
| 53 | H | 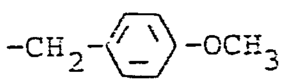 | 135.5-137 |
| 54 | $-CH_3$ | 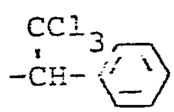 | 156-160 |
| 55 | 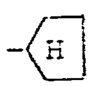 | 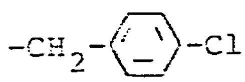 | 183-184 |
| 56 | sec-$C_4H_9$ | 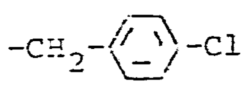 | 154-155 |
| 57 | $-CH_2CH=CH_2$ | $-CH_2CH=CH_2$ | 117-118 |

Tabelle 1 - Fortsetzung

| Verbindung Nr. | $R^1$ | $R^2$ | Physikal. Konstante (Schmp. °C) |
|---|---|---|---|
| 58 | H | $-CH_2C\equiv CH$ | 98-99 |
| 59 | $-CH_2C\equiv CH$ | $-CH_2C\equiv CH$ | 88-90 |
| 60 | $-CH_2CH_2CN$ | $-CH_2CH_2CN$ | 111-112 |
| 61 | $-(CH_2)_7CH_3$ | $-(CH_2)_7CH_3$ | Öl |
| 62 | H | $-CH_2$ 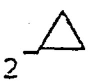 | 99-101 |
| 63 | H | $-CH_2$ 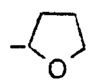 | 91-92 |
| 64 | H | 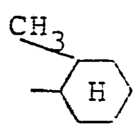 | 205-207 |
| 65 | H | 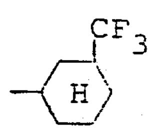 | 159-160 |
| 66 | H | 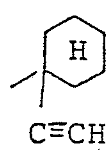 | 159-160 |

Nit-156 -Ausland

Zusätzlich zu den in der Tabelle 1 aufgeführten Verbindungen gemäß der vorliegenden Erfindung wurden auch die folgenden Verbindungen gemäß der vorliegenden Erfindung in ähnlicher Weise synthetisiert:

N-Trichloroacrylnitril-N'-α-naphthylharnstoff,
N-Trichloroacrylnitril-N'-2-pyridylharnstoff,
N-Trichloroacryloyl-N'-cyclopentylharnstoff,
N-Trichloroacryloyl-N'-cycloheptylharnstoff und
N-Trichloroacryloyl-N'-(1,3,5-triazin-2-yl)harnstoff.

Beispiel 2 (Benetzbares Pulver)

15 Teile der Verbindung Nr. 1 der Erfindung, 80 Teile eines Gemisches (1:5) aus pulvriger Diatomeenerde und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf einen pathogenen Pilz und/oder seinen Lebensraum und den Ort, an dem eine Pflanzenkrankheit auftritt, aufgesprüht.

Beispiel 3 (Emulgierbares Konzentrat)

30 Teile der Verbindung Nr. 3 der Erfindung, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf einen pathogenen Pilz und/oder seinen Lebensraum und den Ort, an dem eine Pflanzenkrankheit auftritt, aufgesprüht.

Beispiel 4 (Stäubemittel)

2 Teile der Verbindung Nr. 4 der Erfindung und 98 Teile Tonpulver werden pulverisiert und gemischt, wodurch ein Stäubemittel hergestellt wird. Dieses wird über einem pathogenen Pilz und/oder seinen Lebensraum und den Ort, an dem eine Pflanzenkrankheit auftritt, ausgestreut.

Beispiel 5 (Stäubemittel)

1,5 Teile der Verbindung Nr. 10 der Erfindung, 0,5 Teile Isopropylhydrogenphosphat und 98 Teile Tonpulver werden pulverisiert und gemischt, wodurch ein Stäubemittel hergestellt wird. Dieses wird über einem pathogenen Pilz und/oder seinem Lebensraum und dem Ort, an dem eine Pflanzenkrankheit auftritt, ausgestreut.

Beispiel 6 (Granulat)

Wasser (25 Teile) wird zu einer Mischung aus 10 Teilen der Verbindung Nr. 31 der Erfindung, 30 Teilen Bentonit (Montmorillonit), 58 Teilen Talkum und 2 Teilen Ligninsulfonat zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet und bei 40°C bis 50°C getrocknet, wodurch ein Granulat hergestellt wird. Das Granulat wird über einem pathogenen Pilz und/oder seinem Lebensraum und dem Ort, an dem eine Pflanzenkrankheit auftritt, ausgestreut.

Beispiel 7 (Granulat)

Ein Drehmischer wird mit 95 Teilen Tonmineral-Teilchen mit einer Teilchengrößen-Verteilung im Bereich von 0,2 bis 2 mm beschickt, und unter Drehen des Mischers werden 5 Gew.-Teile der Verbindung Nr. 32 der Erfindung auf die Tonmineral-Teilchen aufgesprüht, so daß diese gleichmäßig benetzt werden. Die feuchte Mischung wird dann bei 40°C bis 50°C getrocknet, wodurch ein Granulat gebildet wird. Das Granulat wird über einem pathogenen Pilz und/oder seinem Lebensraum und dem Ort, an dem eine Pflanzenkrankheit auftritt, ausgestreut.

Beispiel 8

Test der Wirksamkeit gegen die Brusone-Krankheit von Reis durch Wasser-Oberflächen-Anwendung:

Herstellung einer Test-Verbindung:

| | |
|---|---|
| Wirkstoff: | 50 Gew.-Teile; |
| Träger: | 45 Gew.-Teile einer Mischung (1 : 5) aus Diatomeenerde und Kaolin; |
| Emulgator: | 5 Gew.-Teile Polyoxyethylenalkylphenylether. |

Der Wirkstoff, der Träger und der Emulgator wurden in den angegebenen Mengen miteinander vermischt, wodurch ein benetzbares Pulver gebildet wurde. Eine vorher festgelegte Menge des benetzbaren Pulvers wurde mit Wasser verdünnt, wodurch ein Test-Präparat gebildet wurde.

Test-Verfahren

Reispflanzen (Varietät: Asahi) wurden in überfluteten Porzellan-Töpfen mit einem Durchmesser von 12 cm, jeweils drei Stecklinge pro Topf, kultiviert. Im frühen Stadium des Auftretens neuer Triebe der Reispflanzen wurde die Test-Verbindung, die mit vorher festgelegter Konzentration in der oben angegebenen Weise hergestellt worden war, in den angegebenen Dosierungen mittels einer Pipette auf die Wasseroberfläche gegeben, so daß sie nicht in direkte Berührung mit den oberirdischen Teilen der Reispflanzen gelangte. Vier Tage später wurde eine Suspension von Sporen des Pilzes der Brusone-Krankheit (Pyricularia oryzae) auf die Reispflanzen gesprüht, um sie mit dem Pilz zu beimpfen. Die Pflanzen wurden 24 h in einem Inkubator bei einer Temperatur von 23°C bis 25°C und einer relativen Luftfeuchtigkeit von 100 % gehalten. Danach wurden sie in ein Glas-Gewächshaus mit einer Temperatur von 20°C bis 28°C überführt. Sieben Tage nach der Beimpfung wurde der Grad der Erkrankung pro Topf aufgrund des folgenden Bewertungsmaßstabs bestimmt, und der nachstehend definierte Bekämpfungs-Index wurde berechnet.

| Grad der Erkrankung | Fläche der Verletzungen (%) |
|---|---|
| 0 | 0 |
| 0,5 | 2 oder weniger |
| 1 | 3 bis 5 |
| 2 | 6 bis 10 |
| 3 | 11 bis 20 |
| 4 | 21 bis 40 |
| 5 | 41 oder mehr |

$$\text{Bekämpfungs-Index (\%)} = \frac{\text{Grad der Erkrankung einer unbehandelten Fläche} - \text{Grad der Erkrankung einer behandelten Fläche}}{\text{Grad der Erkrankung einer unbehandelten Fläche}} \times 100$$

Die Ergebnisse sind in der Tabelle 2 aufgeführt. Diese Ergebnisse wurden aus drei Töpfen pro Prüfung erhalten.

Tabelle 2

| Beispiel Nr. | Wirkstoff-Konzentration ($g/m^2$) | Bekämpfungs-Index (%) | Phytotoxizität |
|---|---|---|---|
| 1 | 0,5 | 100 | - |
| 3 | 0,5 | 100 | - |
| 4 | 0,5 | 100 | - |
| 10 | 0,5 | 100 | - |
| 14 | 0,5 | 100 | - |
| 19 | 0,5 | 100 | - |
| 22 | 0,5 | 100 | - |
| 31 | 0,5 | 100 | - |
| 32 | 0,5 | 100 | - |
| 37 | 0,5 | 100 | - |
| 41 | 0,5 | 100 | - |
| 53 | 0,5 | 100 | - |
| 58 | 0,5 | 100 | - |
| 60 | 0,5 | 100 | - |
| 63 | 0,5 | 100 | - |
| 66 | 0,5 | 100 | - |
| A (Kontrolle) | 0,51 | 80 | - |

Anmerkungen zu Tabelle A folgende Seite.

Anmerkungen zu Tabelle A:

1) Das Symbol "-" in der Spalte "Phytotoxizität" der Tabelle gibt an, daß keine Phytotoxizität auftrat.
2) Kontrolle A: IBP (allgemeiner Name) /¯S-Benzyldiisopropylphosphorothiolat_7, 17 % Granulat (im Handel erhältlich).

Es wurde sichergestellt, daß auch die anderen, nicht in dem vorstehenden Beispiel erfaßten Verbindungen gemäß der vorliegenden Erfindung eine ausgezeichnete Bekämpfungswirkung gegen die Brusone-Krankheit von Reis zeigen, wenn sie in einer Konzentration des Wirkstoffs von 0,2 bis 1,0 $g/m^2$ auf die Wasseroberfläche aufgebracht werden.

Beispiel 9

Test der Wirksamkeit gegen die Brusone-Krankheit von Reis durch Anwendung auf das Laub:

Test-Verfahren

Reispflanzen (Varietät: Asahi) wurden in unglasierten Töpfen mit einem Durchmesser von 12 cm kultiviert. Im 3- bis 4-Blatt-Stadium wurde eine Verdünnung einer Test-Verbindung, die wie in Beispiel 8 in vorher festgelegter Konzentration hergestellt worden war, auf die Töpfe in einer Aufwandmenge von 50 ml für jeweils 3 Töpfe aufgesprüht. Am nächsten Tage wurde eine Suspension von Sporen des Pilzes der Brusone-Krankheit (Pyricularia oryzae), der künstlich kultiviert worden war, zweimal auf die Reispflanzen gesprüht. Die Pflanzen wurden in einer Kammer bei einer Temperatur von 25°C und einer relativen Luftfeuchtigkeit von 100 % gehalten, um eine Infektion zu bewirken. Die Pflanzen wurden

7 Tage nach der Beimpfung in der gleichen Weise wie in Beispiel 8 geprüft, und der Bekämpfungs-Index (%) wurde berechnet.

Die Ergebnisse sind in der Tabelle 3 aufgeführt.

Tabelle 3

| Beispiel Nr. | Wirkstoff-Konzentration (ppm) | Bekämpfungs-Index (%) | Phyto-toxizität |
|---|---|---|---|
| 1 | 500 | 100 | - |
| 2 | 500 | 100 | - |
| 3 | 500 | 100 | - |
| 10 | 500 | 100 | - |
| 11 | 500 | 100 | - |
| 15 | 500 | 100 | - |
| 17 | 500 | 100 | - |
| 26 | 500 | 100 | - |
| 30 | 500 | 100 | - |
| 32 | 500 | 100 | - |
| 33 | 500 | 100 | - |
| 37 | 500 | 100 | - |
| 38 | 500 | 100 | - |
| 40 | 500 | 100 | - |
| 44 | 500 | 100 | - |
| 45 | 500 | 100 | - |
| 50 | 500 | 100 | - |
| 57 | 500 | 100 | - |
| 62 | 500 | 100 | - |
| 65 | 500 | 100 | - |
| A' (Kontrolle) | 500 | 78 | - |

Anmerkungen zu Tabelle 3:

1) Die Bezeichnung in der Spalte "Phytotoxizität" ist die gleiche wie in der Tabelle 2.

2) A': IBP (allgemeiner Name), 48 % emulgierbares Konzentrat (im Handel erhältlich).

Es wurde sichergestellt, daß auch die anderen, nicht im Vorstehenden beispielhaft erfaßten Verbindungen gemäß der vorliegenden Erfindung eine ausgezeichnete Bekämpfungswirkung gegen die Brusone-Krankheit von Reis zeigen, wenn sie in einer Konzentration des Wirkstoffs von nicht mehr als 500 ppm auf die Blätter und Stengel zur Einwirkung gebracht werden.

Beispiel 10

Test der Wirksamkeit gegen Erkrankungen von Gartenpflanzen:

Die Verbindungen gemäß der vorliegenden Erfindung zeigen eine ausgezeichnete Bekämpfungswirkung gegen Erkrankungen von Gartenpflanzen, wie nachstehend speziell gezeigt wird.

Es wurde sichergestellt, daß beispielsweise die Verbindungen Nr. 4, 17, 21, 22, 30, 31, 32, 33, 38, 41, 42, 43, 50, 57, 63, 65 und 66 ausgezeichnete fungizide Aktivität (Bekämpfungsindex 95 bis 100 %) gegenüber Alternaria brassicae auf "Santosai" (einer Kulturpflanze der Familie der Brassicae) in einer Wirkstoff-Konzentration von 500 ppm aufweisen.

Es wurde sichergestellt, daß beispielsweise die Verbindungen Nr. 4, 8, 12, 21, 33, 38 und 50 der Erfindung

ausgezeichnete fungizide Aktivität (Bekämpfungsindex 95 bis 100 %) gegenüber Colletotrichum lagenarium auf Gurken in einer Wirkstoff-Konzentration von 500 ppm aufweisen.

Es wurde außerdem sichergestellt, daß beispielsweise die Verbindungen Nr. 1, 4, 5, 10, 12, 13, 26, 27, 31, 32, 33, 37, 41, 46, 50 und 63 der Erfindung ausgezeichnete fungizide Aktivität (Bekämpfungsindex 95 bis 100 %) gegenüber Phytophthora infestans auf Tomaten in einer Wirkstoff-Konzentration von 500 ppm aufweisen.

## Patentansprüche

1. Trichloroacryloylharnstoff-Derivat der nachstehenden Formel

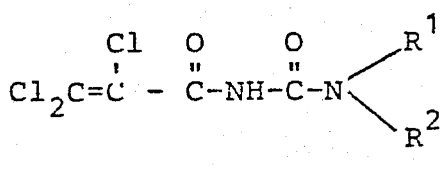

,

in der

$R_1$ für ein Wasserstoff-Atom, eine Alkyl-Gruppe, eine niedere Alkenyl-Gruppe, eine niedere Alkinyl-Gruppe, eine Cycloalkyl-Gruppe oder eine cyano-substituierte niedere Alkyl-Gruppe und

$R_2$ für ein Wasserstoff-Atom, eine Alkyl-Gruppe, eine niedere Alkenyl-Gruppe, eine niedere Alkinyl-Gruppe, eine Cycloalkyl-alkyl-Gruppe, eine cyano-substituierte niedere Alkyl-Gruppe, eine Tetrahydrofurfuryl-Gruppe, eine gegebenenfalls substituierte Cycloalkyl-Gruppe, ein gegebenenfalls substituiertes Aryl, eine gegebenenfalls substituierte Aralkyl-Gruppe oder eine gegebenenfalls substituierte 6-gliedrige heterocyclische Gruppe

stehen.

2. Trichloroacryloylharnstoff-Derivate nach Anspruch 1, wobei in der Formel (I)

$R_1$ für ein Wasserstoff-Atom, eine Alkyl-Gruppe mit 1 bis 8 Kohlenstoff-Atomen, eine niedere Alkenyl-Gruppe mit 2 bis 6 Kohlenstoff-Atomen, eine niedere Alkinyl-Gruppe mit 3 bis 6 Kohlenstoff-Atomen,

eine Cycloalkyl-Gruppe mit 5 bis 7 Kohlenstoff-Atomen oder eine cyano-substituierte niedere Alkyl-Gruppe mit 1 bis 8 Kohlenstoff-Atomen steht und

$R_2$ für ein Wasserstoff-Atom, eine Alkyl-Gruppe mit 1 bis 8 Kohlenstoff-Atomen, eine niedere Alkenyl-Gruppe mit 2 bis 6 Kohlenstoff-Atomen, eine niedere Alkinyl-Gruppe mit 3 bis 6 Kohlenstoff-Atomen, eine Cyclopropylmethyl-Gruppe, eine cyano-substituierte niedere Alkyl-Gruppe mit 1 bis 8 Kohlenstoff-Atomen in der Alkyl-Struktureinheit, eine Tetrahydrofurfuryl-Gruppe, eine Cycloalkyl-Gruppe mit 5 bis 7 Kohlenstoff-Atomen, die substituiert sein kann durch wenigstens einen Substituenten ausgewählt aus der aus niederen Alkyl-Gruppen, Trifluoromethyl-Gruppen und niederen Alkinyl-Gruppen bestehenden Klasse, eine Aryl-Gruppe mit 6 bis 10 Kohlenstoff-Atomen, die substituiert sein kann durch wenigstens einen Substituenten ausgewählt aus der aus Halogen-Atomen, niederen Alkyl-Gruppen, niederen Alkoxy-Gruppen, niederen Alkylthio-Gruppen, Trihalogenomethyl-Gruppen, Cyano-Gruppen, Nitro-Gruppen, Phenyl-Gruppen, Cyclopropyl-Gruppen, Phenoxy-Gruppen, Niederalkylthioniederalkyl-Gruppen und nitro-substituierten Phenylthio-Gruppen, eine Aralkyl-Gruppe mit 6 bis 10 Kohlenstoff-Atomen in dem Aryl-Teil und 1 bis 3 Kohlenstoff-Atomen im Alkyl-Teil, die in dem Aryl-Teil substituiert sein kann durch wenigstens einen Substituenten ausgewählt aus der gleichen Klasse der Substituenten wie im Falle der vorstehenden Aryl-Gruppe und die in dem Alkyl-Teil durch 1 bis 5 Halogen-Atome substituiert sein kann,

oder eine 6-gliedrige heterocyclische Gruppe mit 1 bis 3 Stickstoff-Atomen, die substituiert sein kann durch wenigstens einen Substituenten ausgewählt aus der gleichen Klasse der Substituenten wie im Falle der vorstehenden Aryl-Gruppe, steht.

3. Trichloroacryloylharnstoff-Derivate nach Anspruch 1, wobei in der Formel (I)

$R_1$ für ein Wasserstoff-Atom, eine Alkyl-Gruppe mit 1 bis 8 Kohlenstoff-Atomen, eine niedere Alkenyl-Gruppe mit 2 bis 4 Kohlenstoff-Atomen, eine niedere Alkinyl-Gruppe mit 2 oder 3 Kohlenstoff-Atomen, eine Cycloalkyl-Gruppe mit 5 bis 7 Kohlenstoff-Atomen und eine Cyanoalkyl-Gruppe mit 1 bis 8 Kohlenstoff-Atomen steht und

$R_2$ für ein Wasserstoff-Atom, eine Alkyl-Gruppe mit 1 bis 8 Kohlenstoff-Atomen, eine niedere Alkenyl-Gruppe mit 2 bis 4 Kohlenstoff-Atomen, eine niedere Alkinyl-Gruppe mit 2 oder 3 Kohlenstoff-Atomen, eine Cyclopropylmethyl-Gruppe, eine Cyanoalkyl-Gruppe mit 1 bis 8 Kohlenstoff-Atomen, eine Tetrahydrofurfuryl-Gruppe, eine Cycloalkyl-Gruppe mit 5 bis 7 Kohlenstoff-Atomen, die substituiert sein kann, wobei der Substituent wenigstens ein Glied ausgewählt aus der aus den gleichen niederen Alkyl-Gruppen mit 1 bis 8 Kohlenstoff-Atomen wie vorstehend, einer Trifluoromethyl-Gruppe und den gleichen niederen Alkinyl-Gruppen mit 2 oder 3 Kohlenstoff-Atomen wie vorstehend bestehenden Klasse, Aryl-Gruppen mit 6 bis 10 Kohlenstoff-Atomen, die substituiert sein können, Aralkyl-Gruppen mit 6 bis 10 Kohlenstoff-Atomen in dem Aryl-Teil und 1 bis 3 Kohlenstoff-Atomen im Alkyl-Teil, die substituiert sein können, und

6-gliedrige heterocyclische Gruppen mit 1 bis 3 Stickstoff-Atomen, die substituiert sein können, wobei die Substituenten, die die Aryl-, Aralkyl- oder heterocyclische Gruppe aufweisen können, aus wenigstens einem Substituenten ausgewählt aus der aus Halogen-Atomen, Alkyl-Gruppen mit 1 bis 8 Kohlenstoff-Atomen wie vorstehend, Trihalogenoalkoxy-Gruppen mit 1 bis 8 Kohlenstoff-Atomen, niederen Alkylthio-Gruppen mit 1 bis 8 Kohlenstoff-Atomen, Trihalogenomethyl-Gruppen, einer Cyano-Gruppe, einer Nitro-Gruppe, einer Phenyl-Gruppe, einer Cyclopropyl-Gruppe, einer Phenoxy-Gruppe, Niederalkylthio-niederalkyl-Gruppen mit 1 bis 8 Kohlenstoff-Atomen pro Alkyl-Struktureinheit und einer nitro-substituierten Phenylthio-Gruppe bestehenden Klasse bestehen, wobei der Alkyl-Teil des Aralkyl-Substituenten durch 1 bis 5 Halogen-Atome substituiert sein kann.

4. Trichloroacryloylharnstoff-Derivate nach Anspruch 1, wobei in der Formel (I)

$R_1$ für ein Wasserstoff-Atom, eine Alkyl-Gruppe mit 1 bis 8 Kohlenstoff-Atomen, eine niedere Alkenyl-Gruppe mit 2 bis 4 Kohlenstoff-Atomen, eine niedere Alkinyl-Gruppe mit 2 oder 3 Kohlenstoff-Atomen, Cyclopentyl, Cyclohexyl oder Cycloheptyl oder eine cyano-substituierte Alkyl-Gruppe mit 1 bis 8 Kohlenstoff-Atomen steht und

$R_2$ für ein Wasserstoff-Atom, eine Alkyl-Gruppe mit 1 bis 8 Kohlenstoff-Atomen, eine niedere Alkenyl-Gruppe mit 2 bis 4 Kohlenstoff-Atomen, eine niedere Alkinyl-Gruppe mit 2 oder 3 Kohlenstoff-Atomen, eine Cyclopropylmethyl-Gruppe, eine Cyanoalkyl-Gruppe mit 1 bis 8 Kohlenstoff-Atomen, eine Tetrahydrofurfuryl-Gruppe, Cyclopentyl, Cyclohexyl oder Cycloheptyl, die substituiert sein können, wobei der Substituent wenigstens ein Glied ausgewählt aus der aus niederen Alkyl-Gruppen mit 1 bis 8 Kohlenstoff-Atomen, einer Trifluoromethyl-Gruppe und niederen Alkinyl-Gruppen mit 2 oder 3 Kohlenstoff-Atomen bestehenden Klasse, eine Phenyl- oder Naphthyl-Gruppe, die substituiert sein kann, eine Benzyl- oder Phenethyl-Gruppe, die substituiert sein kann, und eine Pyridyl-, Pyrimidinyl- oder Triazinyl-Gruppe, die substituiert sein kann, wobei die Substituenten, die die Aryl-, Aralkyl- oder heterocyclische Gruppe aufweisen können, aus wenigstens einem Substituenten ausgewählt aus der aus Fluor, Chlor, Brom und Iod, den gleichen niederen Alkyl-Gruppen wie vorstehend, niederen Alkoxy-Gruppen mit den gleichen niederen Alkyl-Gruppen wie vorstehend, niederen Alkylthio-Gruppen mit den gleichen niederen Alkyl-Gruppen wie vorstehend, Trihalogenomethyl-Gruppen mit den gleichen Halogen-Atomen wie vorstehend, einer Cyano-Gruppe, einer Nitro-Gruppe, einer Phenyl-Gruppe, einer Cyclopropyl-Gruppe, einer Phenoxy-Gruppe, Niederalkylthio-niederalkyl-Gruppen mit den gleichen niederen Alkyl-Gruppen wie vorstehend und einer nitro-substituierten Phenylthio-Gruppe bestehenden Klasse bestehen.

5. Trichloroacryloylharnstoff-Derivate nach Anspruch 1, wobei in der Formel (I)

$R_1$ für Wasserstoff, Methyl, Ethyl, Propyl, Isopropyl, n-(iso, sec- oder tert)Butyl, n-Pentyl, n-Hexyl,

n-Heptyl und n-Octyl, Vinyl, Allyl, 1-Propenyl und 1-Butenyl, Ethinyl und 1-Propinyl, Cyclopentyl, Cyclohexyl und Cycloheptyl und eine 2-Cyanoethyl-Gruppe steht und

$R_2$ für Wasserstoff, Alkyl, Alkenyl, Alkinyl und Cyanoalkyl, wie sie vorstehend für $R_1$ definiert wurden, eine Tetrahydrofurfuryl-Gruppe, Cyclopentyl, Cyclohexyl und Cycloheptyl, die durch Methyl, Ethyl, Propyl, Isopropyl, n-(iso, sec- oder tert)-Butyl, n-Pentyl, n-Hexyl, n-Heptyl und n-Octyl, Trifluoromethyl, Ethinyl und 1-Propinyl substituiert sein können, Phenyl, Naphthyl, Benzyl, Phenethyl, Pyridyl, Pyrimidinyl und Triazinyl, die substituiert sein können. wobei die Substituenten, die die Aryl-, Aralkyl- oder heterocyclische Gruppe aufweisen können, aus wenigstens einem Substituenten ausgewählt aus der aus Fluor, Chlor, Brom und Iod, den gleichen niederen Alkyl-Gruppen wie vorstehend, niederen Alkoxy-Gruppen mit den gleichen niederen Alkyl-Gruppen wie vorstehend, niederen Alkylthio-Gruppen mit den gleichen niederen Alkyl-Gruppen wie vorstehend, Trihalogenomethyl-Gruppen mit den gleichen Halogen-Atomen wie vorstehend, einer Cyano-Gruppe, einer Nitro-Gruppe, einer Phenyl-Gruppe, einer Cyclopropyl-Gruppe, einer Phenoxy-Gruppe, Niederalkylthio-niederalkyl-Gruppen mit den gleichen niederen Alkyl-Gruppen wie vorstehend, und einer nitro-substituierten Phenylthio-Gruppe bestehenden Klasse bestehen.

6. Verfahren zur Herstellung eines Trichloroacryloylharnstoff-Derivats der Formel (I),

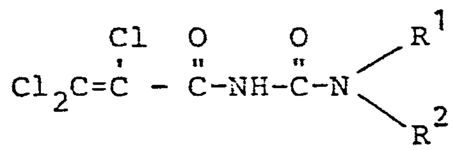

in der

$R_1$ für ein Wasserstoff-Atom, eine Alkyl-Gruppe, eine niedere Alkenyl-Gruppe, eine niedere Alkinyl-Gruppe, eine Cycloalkyl-Gruppe oder eine cyano-substituierte niedere Alkyl-Gruppe und

$R_2$ für ein Wasserstoff-Atom, eine Alkyl-Gruppe, eine niedere Alkenyl-Gruppe, eine niedere Alkinyl-Gruppe, eine Cycloalkyl-alkyl-Gruppe, eine cyano-substituierte niedere Alkyl-Gruppe, eine Tetrahydrofurfuryl-Gruppe, eine gegebenenfalls substituierte Cycloalkyl-Gruppe, eine gegebenenfalls substituierte Aryl-Gruppe, eine gegebenenfalls substituierte Aralkyl-Gruppe oder eine gegebenenfalls substituierte heterocyclische Gruppe

stehen,

dadurch gekennzeichnet, daß Trichloroacryloylisocyanat der nachstehenden Formel (II)

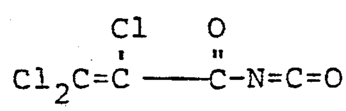

(II)

mit einem Amin der nachstehenden Formel (III)

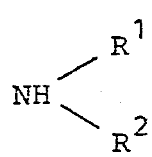

(III)

,

in der $R^1$ und $R^2$ die im Vorstehenden angegebenen Bedeutungen haben, umgesetzt wird.

7. Verwendung von Trichloroacryloylharnstoff-Derivaten der Formel (I) zur Bekämpfung von Pilzen (Fungi).

0118093

8. Verfahren zur Bekämpfung von Pilzen (Fungi), dadurch gekennzeichnet, daß Trichloroacryloylharnstoff-Derivate der Formel (I) auf Pilze und/oder ihre Umwelt zur Einwirkung gebracht werden.

9. Mittel zur Bekämpfung von Pilzen (Fungi), enthaltend wenigstens eine Verbindung nach Anspruch 1 bis 5.

10. Verfahren zur Herstellung von Mitteln zur Bekämpfung von Pilzen (Fungi), dadurch gekennzeichnet, daß Trichloroacryloylharnstoff-Derivate der Formel (I) mit Streckmitteln und/oder oberflächenaktiven Mitteln vermischt werden.

Europäisches Patentamt

# EUROPÄISCHER RECHERCHENBERICHT

0118093
Nummer der Anmeldung

EP 84 10 2049

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | FR-A-2 304 603 (BAYER)<br>* Ansprüche *<br>--- | 1,6,7 | C 07 C 127/22<br>A 01 N 47/34<br>C 07 D 213/75<br>C 07 D 239/42<br>C 07 D 251/46<br>C 07 D 251/22<br>C 07 D 307/14 |
| A | FR-A-2 265 742 (BAYER)<br>* Seiten 3,4 *<br>--- | 1 | |
| A | DE-C- 888 316 (CASSELLA FARBWERKE MAINKUR AG)<br>* Seite 1 *<br>----- | 1 | |
| | | | RECHERCHIERTE SACHGEBIETE (Int. Cl. 3) |
| | | | C 07 C 127/00 |

Der vorliegende Recherchenbericht wurde für alle Patentansprüche erstellt.

| Recherchenort | Abschlußdatum der Recherche | Prüfer |
|---|---|---|
| DEN HAAG | 15-05-1984 | BONNEVALLE E.I.H. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503. 03.82